(19) Europäisches Patentamt
European Patent Office
Office européen des brevets

(11) Publication number: **0 364 941 B1**

(12) # EUROPEAN PATENT SPECIFICATION

(45) Date of publication of patent specification: **15.02.95**

(51) Int. Cl.[6]: **C07D 295/088**, A61K 31/445

(21) Application number: **89119232.0**

(22) Date of filing: **17.10.89**

The file contains technical information submitted after the application was filed and not included in this specification

(54) **Branched-chain alkyl esters of 2-(4-(2-piperidino ethoxy)benzoyl)benzoic acid, processes for their preparation and their use as spasmolytic agents.**

(30) Priority: **21.10.88 EP 88117521**

(43) Date of publication of application:
**25.04.90 Bulletin 90/17**

(45) Publication of the grant of the patent:
**15.02.95 Bulletin 95/07**

(84) Designated Contracting States:
**AT BE CH DE ES FR GB GR IT LI LU NL SE**

(56) References cited:
**FR-A- 1 057 372**

(73) Proprietor: **HOECHST AKTIENGESELLSCHAFT**

**D-65926 Frankfurt (DE)**

(72) Inventor: **Bal-Tembe, Swati, Dr.**
**H-14, Hoechst Staff Ouarters**
**Darga Road**
**Mulund**
**Bombay 400 082 (IN)**
Inventor: **Blumbach, Jürgen, Dr.**
**Nepean Sea Road**
**"Nilandri"**
**Bombay 400 006 (IN)**
Inventor: **Dohadwalla, Alihussein Nomanbhai, Dr.**
**Noman Mansion**
**139 C, Cumballa Hill**
**August Kranti Marg**
**Bombay 400 036 (IN)**
Inventor: **Lal, Bansi, Dr.**
**30A, Advani Apartments**
**Mulund (West)**
**Bombay 400 080 (IN)**
Inventor: **Punekar, Narayan Sudhindra, Dr.**
**No. 6, Anand Niwas**
**Behind RPD**
**Tilakwadi**
**Belgaum 590 006 (IN)**
Inventor: **Rajgopalan, Ramanujam, Dr.**
**203, B Wing**
**Arthi**
**Sarojini Naidu Road**
**Mulund (West)**
**Bombay 400 080 (IN)**
Inventor: **Rupp, Richard Helmut, Dr.**
**Roederweg 16a**
**D-6240 Königstein/Taunus (DE)**

EP 0 364 941 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Bickel, Martin, Dr.**
**Mittelstedter Weg 3**
**D-6380 Bad Homburg (DE)**

## Description

The present invention relates to isopropyl-2-[4-(2-piperidino-ethoxy)-benzoyl]-benzoic acid and pharmaceutically acceptable salts thereof, processes for its preparation, and its use as spasmolytic agents in medicaments.

Esters of 2-[4-(2-piperidino-ethoxy)-benzoyl]-benzoic acid of the formula I ($R_1$ = lower alkyl) are described in U.S. Patent 2,681,340, to be antispasmodic agents, wherein the meaning of $R_1$ as a lower alkyl group has a very limited definition. In the said definition, although R1 includes lower alkyl groups, the specification only describes the preparation of straight-chain methyl, ethyl and n-butyl esters, and cites in the claims as particular compounds only the methyl and ethyl esters as the compounds of choice.

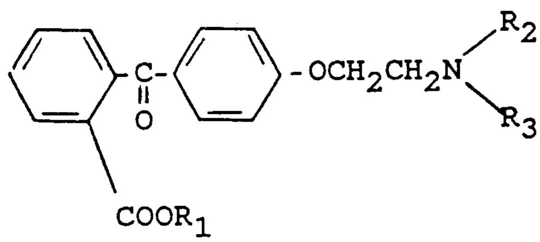

I

To date, no branched-chain esters of the formula I are known.

Pitofenone (formula II) is a representative compound of

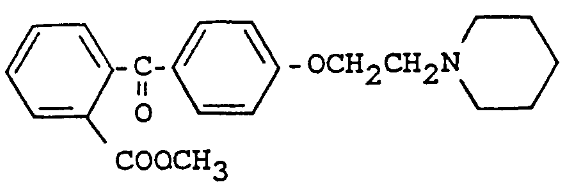

II

the series described in the above cited U.S. Patent 2,681,340 and is used alone or in combination with metamizole in antispasmodic preparations like Baralgan[(R)].When pitofenone is administered intravenously, it displays potent antispasmodic activity. When it is, however, administered orally, the activity is considerably reduced, which is attributed to rapid metabolism of the methyl ester to the corresponding acid.

It would be highly advantageous to have an orally active antispasmodic preparation.

The present invention describes novel branched-chain alkyl ester isopropyl-2-[4-(2-piperidino-ethoxy)-benzoyl]-benzoate, which is surprisingly not only more potent spasmolytic agent than pitofenone and the compounds of the class described in U.S. Patent 2,681,340, but also more stable to hydrolysis by enzymes, longer acting, and metabolically more stable when administered enterally or parenterally to mammals. These properties contribute to making the compound of the invention specially useful for medicaments that can be orally administered as spasmolytic agents.

The present invention relates to the compound represented by formula III,

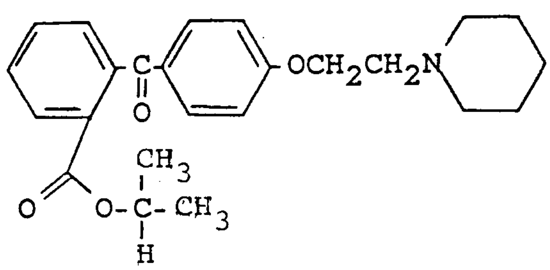

III

and its pharmaceutically acceptable salts.

The invention further relates to processes for the preparation the compound of fomula III. Such a compound can be obtained by various methods.

A carboxylic acid of formula IV (R = H), for instance, can be derivatised at the carboxyl group by known methods [E. Haslam, Protective Groups in Organic Chemistry (J. F. W. McOmie Ed.), p.183. Plenum Press, London (1973), and E. Haslam, Tetrahedron, 1980, 36, 2409, and references cited therein], and the resulting phenolic ester of formula IV in which R is represented

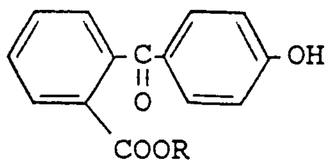

IV

by formula V

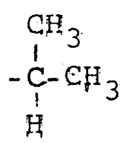

V

can then be treated with a 2-piperidino-ethyl derivative bearing a good leaving group, or its- corresponding salt, for instance, a 2-piperidino-ethyl halide, for example, 2-piperidino-ethyl chloride or 2-piperidinoethyl chloride hydrochloride in the presence of a base to obtain the compound of the invention of formula III.

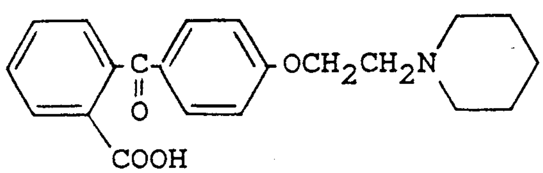

VI

Alternatively, an acid of the formula VI can directly be esterified with the appropriate alcohol in presence of a condensing agent by known methods cited above.

Appropriate condensing agents are for example conc. $H_2SO_4$, trifluoroacetic anhydride and thionyl chloride.

In another method, the carboxylate salt of the acid of formula VI can be treated by known methods (loc. cit.) with appropriate alkyl halides to form compounds of formula III.

These reactions can be performed in some cases neat, or in organic solvents or mixtures thereof not interfering with the reaction, such as for example ketones, like acetone or butan-2-one, N,N-disubstituted amides, like N,N-dimethylformamide or N,N-dimethylacetamide, halogenated hydrocarbons, like carbon tetrachloride, chloroform, methylene chloride, ethers like diethyl ether, diisopropylether or methyl-tert.-butyl ether, aromatic hydrocarbons, like benzene, toluene or xylene, esters like ethyl acetate or butyl acetate, dimethylsulfoxide or hexamethyl phosphoric triamide. Also mixtures of these solvents with water can be used. When phase transfer catalysts are used, catalysts like methyltrioctylammonium chloride (for example Aliquat$^{(R)}$ 336) or tetrabutylammonium bromide are suitable.

Furthermore, under appropriate conditions, the reactant alcohol may also be used as a solvent.

The reactions can be carried out at temperatures between -20 °C and the boiling point of the solvent used.

For the conversion of compound IV, in which R is equal to formula V, to compound III, a base may be added to shorten the reaction time. As base may be considered a metal hydroxide like sodium hydroxide, potassium hydroxide, calcium hydroxide or magnesium hydroxide, a metal carbonate, like sodium carbonate or potassium carbonate, a bicarbonate, like sodium bicarbonate or potassium bicarbonate, an alcoholate like sodium methoxide or sodium ethoxide, or an organic base like tertiary amines such as triethylamine, N,N-diethylaniline or pyridine.

The compound of the invention of formula III may exist as a free base or in the form of a pharmaceutically acceptable salt with an organic or inorganic acid such as hydrochloric acid, sulfuric acid, acetic acid, malonic acid, maleic acid, tartaric acid or citric acid. Preferred is the salt with hydrochloric acid.

The compound of the invention displays the following special properties :

1) Low esterase hydrolysis rates

2) Potent and long lasting antispasmodic action in vitro and in vivo.

3) Increased bioavailability on oral administration to mammals as demonstrated in the experiments described below by using the following test compounds :

Test Compound I :
Methyl-2-[4-(2-piperidino-ethoxy)-benzoyl]-benzoate hydrochloride (Pitofenone)
Test Compound II :
Isopropyl-2-[4-(2-piperidino-ethoxy)-benzoyl]-benzoate hydrochloride.
Test Compound III :
2-[4-(2-piperidino-ethoxy)-benzoyl]-benzoic acid.

Experiment 1 -

Enzymatic hydrolysis rates for test compounds :

Porcine liver esterase (0.5 U = 2 µg) was preincubated in 40 mM Tris-HCl buffer, pH 8.0, at 37°C for 5 min. The reaction was initiated with the addition of the test compound (final concentration, 1 mM) in the final volume of 250 µl. Enzyme reaction was terminated at different time intervals by the addition of 60 µl of 1 M acetic acid. The solution was made alkaline (pH 10.5) by adding 0.4 M NaOH and the alkaline solution was rapidly extracted (three times) with 2.5 ml of chloroform. An aliquot of the aqueous supernatant was diluted with double distilled water and the absorbance was measured at 290 nm. Amount of test compound III (molar extinction = 14,400) formed was calculated. Results are shown in Table I.

Table I

| Time (hours) | µmol test compound III formed per mg protein per ml | |
|---|---|---|
| | Test Compound | |
| | I | II |
| 0.0 | 0.00 | 0.00 |
| 0.5 | 0.50 | 0.05 |
| 2.0 | 1.62 | 0.10 |
| 3.5 | 2.78 | 0.10 |

Experiment 2 -

Antispasmodic Activity in isolated guinea pig ileum model:

Small intestines obtained from freshly sacrificed guinea pigs (weight range, 200 - 400 g) of either sex were cleaned and stored in tyrode solution. A piece of ileum was mounted in an organ bath containing tyrode solution at 37°C and maintained at a tension of 0.5 to 1.0 g. The solution was continuously aerated with compressed air. After an initial equilibration period, a sub-maximal dose of acetylcholine required for measurable contraction was standardised. Tension changes were monitored using an isotonic strain gauge attached to the tissue and the responses were recorded on a strip chart recorder.

Antispasmodic activity of test compounds at different concentrations was followed and the $IC_{50}$ values were calculated from dose response curves. Results are shown in Table 2.

Table 2

| Test Compound | R Group | Guinea pig ileum | |
|---|---|---|---|
| | | $IC_{50}$ (µg/ml) | Duration of action (min) |
| I | $-CH_3$ | 1.00 | < 1.5 |
| II | $-CH(CH_3)_2$ | 0.01 | > 7.0 |

Experiment 3

Antispasmodic activity in anaesthetized dog:

Method:

Male mongrel dogs of weight range 10 to 15 kg were anaesthetised with pentobarbitone sodium (35 mg/ kg,i.v.). Slow i.v. infusion of pentobarbitone (5 mg/kg/hour) was given for maintenance anaesthesia. The endotracheal tube was inserted to facilitate spontaneous breathing. Both femoral artery and vein were cannulated respectively for recording blood pressure and administering drugs. After laparotomy, a small portion of small intestine distal to duodenum was isolated and saline filled balloon inserted and the opening was sutured. A fine polyethylene cannula was then inserted into the mesenteric artery which supplies to the area of intestine containing balloon for injection of carbachol or acetylcholine. A Statham pressure transducer was then attached to balloon cannula for recording both circular and longitudinal muscle contractions. All the parameters were recorded on 4 channel Nihon-Kohden recorder. The intestinal contractions were recorded after administering a standard dose of carbachol or acetylcholine (0.5 to 3 µg).

Intra-duodenal administration:

Test compounds I and II were given at different doses 3,10 and 20 mg/kg intra-duodenally and spasmolytic activity was assessed at various time intervals.

Anti-spasmodic activity was assessed by calculating percent reduction in agonist induced contractions and also noting onset and duration of activity. The test compounds were dissolved in distilled water (1% soln.). Results see Table 3.

Table 3

| Spasmolytic activity after intra-duodenal administration in anaesthetised dog: | | | | |
|---|---|---|---|---|
| Test compounds | Dose mg/kg | Percent inhibition | On-set (min) | Duration (min) |
| Test compd. II | 3 | 54 | 7 | 35 |
| | 10 | 83 | 6.5 | 78 |
| Test compd.I | 10 | N.A. | N.A. | N.A. |
| | 20 | 63 | 20 | 40 |
| N.A. Not active. | | | | |

Intravenous administration:

Test compounds I and II were given at different doses (10, 30, 100, 300 and 1000 µg/kg) intravenously and spasmolytic activity was assessed at different time intervals.

Anti-spasmodic activity was assessed by calculating percent reduction in agonist induced contractions. The $ID_{50}$ value was calculated from the dose response curve. Results see Table 4.

Table 4

| Spasmolytic activity after intravenous administration in anaesthetised dog: | | |
|---|---|---|
| Test compounds | $ID_{50}$ (mg/kg,i.v.) | Duration of action (min) |
| Test compd. I<br>Test compd. II | 0.140<br>0.010 | 10 - 30<br>> 60 |

Experiment 4

Method:

Ref.: J.M.A. Zwagemakers and V. Claassen, Arzneim-Forsch/Drug Res. 30 (II) Nr.9, p.1517 (1980).

The test compounds I and II were given orally by gavage in a range of 10 to 100 mg/kg doses 30 min before charcoal suspension (0.2 ml/animal, 10% charcoal in 5% gum acacia) in 18 hrs fasted mice (male or female, weight range 18 to 25 g). 30 min after administration of charcoal animals were killed and the extent of charcoal propulsion in the small intestine was measured. The inhibitory activity of test compound on charcoal transport was assessed as percent inhibition compared to length of intestine. For control group 10 ml/kg of saline was given. Results Table 5.

Table 5

| Effect on gastrointestinal propulsion in mice : | | | |
|---|---|---|---|
| Test compds | Dose mg/kg.p.o | Percent Inhibition | No. of animals |
| Control | 10 ml/kg (vehicle) | 12.7 ± 1.2 | 40 |
| Test compd. II | 10<br>30<br>60 | 38.0 ± 4.0<br>41.0 ± 3.0<br>46.0 ± 5.0* | 20<br>20<br>20 |
| Test compd. I | 60<br>100 | 24.0 ± 3.0<br>30.0 ± 4.3 | 16<br>13 |

* P = < 0.001 compared with 60 mg/kg of pitofenone (unpaired 't' test)

Experiment 5

Effects of Test Compound II (isopropyl-2-[4-(2-piperidino-ethoxy)-benzoyl]-benzoate hydrochloride) on the Motility of the small Intestine in the conscious Dog:

Methods:

Male dogs (THG Tierhandelsgesellschaft Rodenbach, Hoe:BEAK (Beagle)) weighing 15 - 20 kg were used in all experiments. At least 4 weeks prior to the experiment the dogs had been equipped with miniaturized strain gage force transducers (T1-T3) (2x4 mm), sutured onto the gut at the locations mentioned below. The cables of the transducers were embedded into a cannula which carried the plug for external electric connection. This stainless steel cannula was implanted into the abdominal wall, close below the costal arch. Each transducer was connected to a measuring bridge. The signals were on line stored using a HP 9835 A computer, allowing later analysis of the data.

T1 : Proximal jejunum
T2 : Distal jejunum
T3 : Ileum

18 hours prior to the experiment food was withdrawn from the animals, water ad libidum. All experiments started between 08:00 and 09:00 am.

The following motility parameters were determined:
The integral under the concentration curves at 10 min intervals (mNx10min) as an index of phase I + II activity.
The duration of drug action (min.)
The duration of the interdigestive migrating complex (IMC) (min) as a measure of phase III activity.

Treatments:

Test Compound II was given at doses of 0.2 and 0.5 mg/kg i.v. or 5 mg/kg i.g., dissolved in saline, in a volume of 1 ml/kg (i.v.) or 2 ml/kg (i.g.), n = 3 - 6. Control n = 26.
Reference substances: Buscopan, pitofenon.
Buscopan was given at doses of 0.3 and 1 mg/kg i.v., dissolved in saline, in a volume of 1 ml/kg, n = 4 - 6. Pitofenone was given at doses of 1 mg/kg i.v., dissolved in saline, in a volume of 1 ml/kg, n = 8.

Statistical methods:

$ID_{50}$-values were determined graphically.

Results: Tables 6 - 8, Figures 1 - 2.

Summary:

All three compounds inhibited the spontaneous motility of the small intestine in the conscious dog (Tables 6 - 7). The rank order of potency was:
Test Compound II > buscopan > pitofenon
The intravenous $IC_{50}$-values determined from maximal inhibition (Table 7) were:

| | |
|---|---|
| Test Compound II : | 220 µg/kg |
| Buscopan : | 600 µg/kg |
| Pitofenon : | 1000 µg/kg |

The inhibitory effect of an i.v.-injection of 0.5 mg/kg of test compound II lasted for approximately one hour, whereas buscopan at a dose of 1 mg/kg i.v. had only a duration of action of 30 min (Table 6) .
When test compound II was given intragastrically, 5 mg/kg, a pronounced inhibition of phase III activity could be monitored (Table 8, Fig. 1,2).

**Table 6** Effect of small intestinal motility (Phase I and II activity)
(Area under the contraction curve in mNx10min as a measure for gut motility for phase I and II)

| | Dose | | Predrug | | | Postdrug mN x 10 min | | | |
|---|---|---|---|---|---|---|---|---|---|
| | mg/kg | N | -20 - 0 | 10 | 20 | 30 | 40 | 50 | 60 min |
| Test compound II | 0.2 | 4 | 2.15 | 1.54 | 1.42 | 1.88 | 1.77 | 1.78 | 2.19 |
| | | | ±0.70 | ±0.36 | ±0.52 | ±1.04 | ±0.86 | ±0.68 | ±1.13 |
| | 0.5 | 6 | 2.62 | 0.54 | 0.50 | 0.99 | 1.17 | 1.61 | 2.46 |
| | | | ±1.39 | ±0.69 | ±0.57 | ±0.80 | ±0.73 | ±1.16 | ±2.23 |
| Pitofenon | 1 | 8 | 2.96 | 1.58 | 2.01 | 2.59 | 2.29 | 3.16 | 3.64 |
| | | | ±2.0 | ±1.84 | ±1.45 | ±1.59 | ±1.92 | ±1.12 | ±2.42 |
| Buscopan | 0.3 | 6 | 2.09 | 1.78 | 1.74 | 2.17 | 2.15 | 1.80 | 1.60 |
| | | | ±0.81 | ±0.86 | ±1.28 | ±0.98 | ±0.69 | ±0.67 | ±0.82 |
| | 1 | 4 | 2.03 | 0.85 | 0.86 | 1.23 | 2.03 | 2.09 | 1.99 |
| | | | ±0.49 | ±0.57 | ±0.53 | ±0.21 | ±1.10 | ±0.89 | ±0.70 |

Results show means ± SD

**Table 7** Effect on small intestinal motility in the conscious dog (Phase I+II activity) i.v. (Area under the concentration curve in mN·10min as a measure for gut motility for phase I and II)

| Treatment | Dose mg/kg | Inhibition Maximal % | Inhibition Mean % | Duration [a] of action min | $ID_{50}$ mg/kg |
|---|---|---|---|---|---|
| Test compound II | 0.2 | 34 | 22 ± 4 | 50 | 0.26 |
| | 0.5 | 81 | 54 ± 11 | 60 | |
| Pitofenon | 1 | 47 | 29 ± 7 | 40 | 1 |
| Buscopan | 0.3 | 29 | 23 ± 6 | 20 | 0.6 |
| | 1 | 58 | 52 ± 6 | 30 | |

Results show means ± SD

a) = when postdrug values reached 90 % or more of predrug values

**Table 8** Effect on small intestinal moitility in the conscious dog (Phase III activity) Intragastrical application

| Treatment | Dose mg/kg | N | Duration of cycles min |
|---|---|---|---|
| Control | -- | 26 | 121 ± 7 |
| Test compound II | 5 | 3 | 298 ± 18* |

Results show means ± SD
* $p < 0.05$ vs. control

Experiment 6

Effects on the Motility of the large Bowel in conscious Dogs

Methods:

Male dogs (THG Tierhandelsgesellschaft Rodenbach, Hoe:BEAK (Beagle)) weighing 15 - 20 kg were used in all experiments.

At least 4 weeks prior the experiment the dogs had been equipped with miniaturised strain gage force transducers (T1-T2) (2x4mm), sutured onto the gut at the locations mentioned below. The cables of the transducers were embedded into a cannula which carried the plug for external electric connection. This stainless steel cannula was implanted into the abdominal wall, close below the costal arch. Each transducer was connected to a measuring bridge. The signals were on line stored using a HP 9835 A computer, allowing later analysis of the data.

T1 : Colon ascendens 10 cm aborafrom the ileoceval valve (ICV)
T2 : Colon transversum 25 cm aborad from the ICV

18 hours prior to the experiment food was withdrawn from the animals, water ad libidum. All experiments started between 08:00 and 09:00 am.

The following parameters were determined:
The duration of the cycle (min)
The duration of the colonic motor complex (CMC) (min)
The maximal height of the concentrations (AU: arbitrary units)
Data from the two recording sites (T1-T2) were pooled.

Treatments:

Test compound II was given at a dose of 3 mg/kg i.g., dissolved in saline, in a volume of 2 ml/kg, n = 5, control n = 23.

Statistical methods: For detection of significant differences ($p < 0.05$) the unpaired t-test was used. Only significant differences were indicated by *.

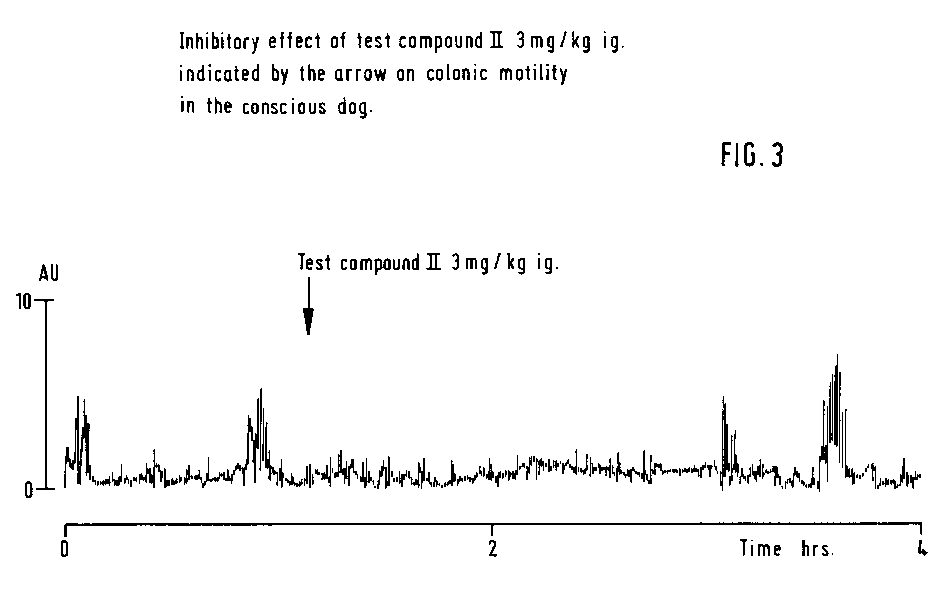

Results: Table 9, Figure 3

**Table 9**

Effects on the colonic motor complex (CMC) of the large bowel in the conscious dog.

Route of administration: intragastrically

| Treatment | Dose mg/kg | N[a] | N[b] | Duration Cycle min | Duration CMC min | Max.heigth of CMC AU | Total number of spikes n/CMC |
|---|---|---|---|---|---|---|---|
| Control | -- | 28 | 112 | 39±10 | 10±3 | 50±25 | 5.5±1.7 |
| Test compound II | 1 | 4 | 4 | 28±11 | 6±2 | 50±25 | 5.6±1.7 |
| | 3 | 14 | 14 | 136±42* | 11±3 | 40±23 | 5.5±1.0 |
| Buscopan | 3 | 6 | 6 | 35±19 | 6±4 | 35±17 | ND |
| | 10 | 4 | 4 | 32± 5 | 5±1 | 35±10 | ND |

a: Number of experiments

b: Number of cycles analysed

ND:not dertermined

Summary:

Test compound II, 4 mg/kg i.g., inhibited colonic motility (CMC in the conscious dog completely for about 2 hours. 1 mg/kg i.g. was uneffective. Buscopan at doses of 3 and 10 mg/kg i.g. had no effect on colonic motility in the conscious dog.

The compounds of the invention and their salts possess valuable spasmolytic properties and are suitable for the treatment of all types of spasms, mild and acute. The compounds of this invention and their salts can also be used in combination with other pharmacologically active substances, for example, antiinflammatory, analgesic, anti-anxiety, CNS depressant agents and other such therapeutic agents that are pharmacologically acceptable to be used in combination with spasmolytic agents.

The compounds of the invention and their physiologically tolerable salts can be administered orally, parenterally, (intramuscularly, intravenously, subcutaneously) rectally, or topically, optionally in the form of an aerosol.

The compounds of the invention and their physiologically tolerable salts can be administered either per se or in admixture or conjunction with a pharmaceutically suitable carrier material. For oral administration the active compounds may be admixed with the carrier and transformed into the usual form for administration, for example, tablets, push-fit capsules, aqueous alcoholic or oily suspensions or solutions. Suitable carrier materials are, for example, magnesium carbonate, milk sugar, maize starch and magnesium stearate. The compositions can be prepared in the form of dry or moist granules. An oily carrier or solvents may be a vegetable or animal oil, for example, sunflower oil or cod-liver oil.

The active compounds may be administered intravenously. To this end, a compound of the invention or a physiologically tolerable salt thereof, as far as it has sufficient solubility, is generally dissolved in one of the usual auxiliaries, which may also act as a dissolving intermediary or buffer.

The solvents for intravenous administration are, for example, water, physiological sodium chloride solution and dilute alcohols, for example, ethanol, propanediol and glycerol; furthermore, sugar solutions, for

example, glucose and mannitol solutions, or a mixture of two or more of the aforesaid solvents.

The pharmaceutical preparations are preferably in unit dosage form.

The following examples illustrate the invention but do not restrict the scope of the invention.

EXAMPLE I

Isopropyl-2-[4-(2-piperidino-ethoxy)-benzoyl]-benzoate

Method I :

To 2-(4-hydroxy-benzoyl)-benzoic acid (60 g, 0.25 mol) were added 2-propanol (1020 ml) and concentrated $H_2SO_4$ (25 ml) and the reaction mixture was refluxed for 24 hours. 2-Propanol then was distilled off under vacuum. The residue was dissolved in ethyl acetate (1000 ml), cooled and washed with sodium bicarbonate solution (2 x 500 ml) followed by water (750 ml), dried and concentrated to give 70 g of isopropyl ester as a dark oil, which could be crystallised from diisopropyl ether to give white crystals, mp. 95 - 97 ° C.

A mixture of the isopropyl ester (28.4 g, 0.1 mol), 2-piperidinoethyl chloride (23.6 g, 0.16 mol) and anhydrous $K_2CO_3$ (57 g, 0.38 mol) in dry 2-butanone (1000 ml) was refluxed for 6 hours. The solvent was distilled off under vacuum, the mixture poured into water (800 ml) and extracted with ethyl acetate (1.1 lit). The ethyl acetate layer was washed with 1% aqueous KOH solution, followed by water (2 x 500 ml), dried over anhydrous $Na_2SO_4$ and concentrated to give 36 g of a dark viscous oil. Flash chromatography (silica gel, $CHCl_3$-EtOAc-$CH_3OH$, 74:20:6) gave 33 g (83%) of the title compound as a viscous oil, which could be crystallised from n-pentane at ca. -6 ° C to give white crystals, mp 34 - 35 ° C.

Alternatively a mixture of the isopropylester (28.4 g, 0.1 mole), 2-piperidinoethyl chloride hydrochloride (25.9 g, 0.14 mole), anhydrous $K_2CO_3$ (38.7 g, 0.28 mole) in 2-butanone (215 ml) containing water (9.5 ml) was refluxed for 3 hours. After workup as described above, 34.5 g of the title compound were obtained in 87 % yield.

IR (neat) : 3000, 2950, 1720, 1670, 1600 $cm^{-1}$,

NMR ($CDCl_3$) : $\delta$ 7.92 - 8.02 (m, 1H), 7.19 - 7.7(m, 5H) 6.72 - 6.92 (m, 2H), 4.96 (h, 1H, 5.8Hz), 4.12 (t, 2H, 6.5 Hz), 2.78 (t, 2H, 6.5Hz), 2.40 - 2.60 (m, 4H), 1.40 - 1.75(m, 6H), 1.06 (d, 6H, 5.8 Hz)

Method II

A mixture of 2-[4-(2-piperidino-ethoxy)-benzoyl]-benzoic acid (14.8 g, 0.04 mol), dry 2-propanol (300 ml) and 14.8 ml concentrated $H_2SO_4$ was refluxed for 9 hours. 2-propanol was distilled off in vacuo. Aqueous potassium carbonate was added to the cooled reaction mixture and it was extracted with ether (3 x 200 ml).

The ether layer was washed with water, dried and concentrated to give 10.8 g of an oil. Flash chromatography (silica gel, $CHCl_3$ - EtOAc - $CH_3OH$, 74:20:6) gave 6.7 g (41%) of the title compound as a viscous oil, identical in its physical properties with product of example 1, method I.

Method III :

2-[4-(2-Piperidino-ethoxy)-benzoyl]-benzoic acid (23.4 g, 0.07 mol) was suspended in 2-propanol (400 ml) at -5 ° C. Thionyl chloride (67 ml) was added dropwise over a thirty minute period maintaining the temperature between -5 to -10 ° C. The clear solution was refluxed for 4.5 hours and cooled. Thionyl chloride and 2-propanol were distilled off under vacuum. To the reaction mixture was added a saturated solution of $NaHCO_3$ until the pH reached 8 and the mixture extracted with ether (250 ml x 3). The ether layer was washed with water (200 ml x 2), brine (200 ml x 2), dried over anhydrous $Na_2SO_4$ and concentrated to give 21.4 g of a crude oil. Flash chromatography (silica gel, $CHCl_3$ -EtOAc-MeOH) gave 12.1 g (46%) of the title compound as a viscous oil identical in its physical properties with product of Example 1, Method I.

Method IV:

A mixture of 2-[4-(2-piperidino-ethoxy)-benzoyl]-benzoic acid (1.05 g, 0.003 mol), 20 ml HMPA and a solution of NaOH (0.18 g, 0.0045 mol) in 1.5 ml water was stirred for 30 min and 2-bromopropane (1.22 ml, 0.012 mol) was added. The reaction mixture was stirred at room temperature for 24 hours. It was then poured into water (150 ml) and extracted with ethyl acetate. The ethyl acetate layer was washed several

times with water, dried over anhydrous $Na_2SO_4$ and concentrated in vacuo to give 1.3 g of a viscous oil. Flash chromatography (silica gel, $CHCl_3$ - $CH_3OH$) gave 0.72 g (62%) of the title compound as an oil, identical in its physical properties with product of example 1, Method I.

Method V:

To a mixture of 2-bromopropane (0.62 g, 5 mmol) and Aliquat 336 (0.22 g, 0.54 mmol) was added dry powdered potassium salt of 2-[4-(2-piperidino-ethoxy)-benzoyl]-benzoic acid (2.15 g, 5.4 mmol). The flask was fitted with a $CaCl_2$ guard tube and shaken for 15 mins. It was then filtered with a reflux condenser and the reaction mixture heated at 60 ° C (bath temperature) for 25 hours. The product was triturated with 25 % EtOAc-petroleum ether (25 ml) and filtered over a bed of neutral alumina (40 g, grade I) followed by elution with 25 % EtOAc-petroleum ether (150 ml). The combined filtrate was concentrated to give 1.5 g (75 %) of the title compound as a colourless viscous oil, identical in its physical properties with product of Example 1, Method I.

EXAMPLE 2

Isopropyl-2-[4-(2-piperidino-ethoxy)-benzoyl]-benzoate hydrochloride

5.54 g of isopropyl-2-[4-(2-piperidino-ethoxy)-benzoyl]-benzoate was dissolved in 40 ml dry $CH_2Cl_2$. Ethereal HCl was added dropwise until the pH was between 2 and 3. Excess HCl was removed on a steam bath. The solvent was distilled off in vacuo to give a sticky brown residue which was crystallised from EtOH-$Et_2O$. The resulting white crystals of the title compound were filtered and washed with 2% EtOH-$Et_2O$.

Yield : 3.31 g (58%)
mp 110 ° - 112 ° C; Anal. Calcd. for $C_{24}H_{30}ClNO_4$ :
C, 65.37; H, 7.09; N, 3.18; Cl, 8.04.
Found : C, 65.62; H, 7.13; N, 3.16; Cl, 8.38.

## Claims

**Claims for the following Contracting States : AT, BE, CH, DE, FR, GB, IT, LI, LU, NL, SE**

1. Isopropyl-2-[4-(2-piperidino-ethoxy)-benzoyl]-benzoate and its pharmaceutically acceptable salts.

2. A process for the production of the compound as claimed in claim 1, wherein a compound of the formula IV

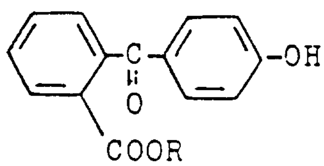

IV

in which R is represented by formula V

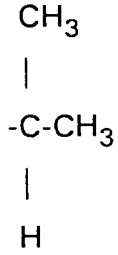

V

is treated with a 2-piperidinoethyl derivative bearing a good leaving group.

3. A process for the production of compounds as claimed in claim 1, wherein a compound of the formula VI

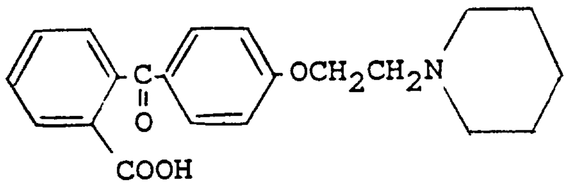

VI

is esterified with an alcohol of the formula VII

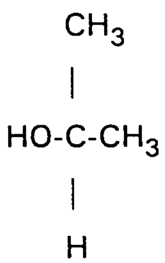

VII

4. A process for the production of compounds as claimed in claim 1, wherein the carboxylate salt of the acid of formula VI is reacted with an alkyl halide.

5. A pharmaceutical preparation containing the compound as claimed in claim 1, optionally besides customary excipients and/or auxiliaries.

6. A pharmaceutical preparation as claimed in claim 5 for oral administration.

7. The use of the compound as claimed in claim 1 for the production of a pharmaceutical agent with spasmolytic activity.

8. The compound as claimed in claim 1 as a pharmaceutical.

**Claims for the following Contracting States : ES, GR**

1. A process for the production of a compound of the formula III

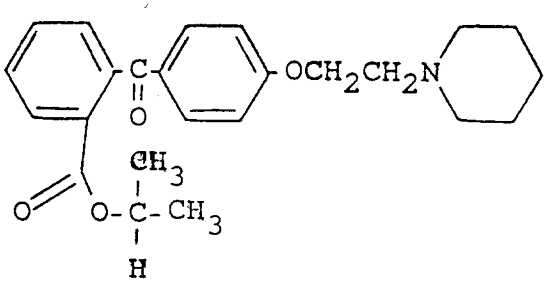

III

and its pharmaceutically acceptable salts, wherein a compound of the formula IV

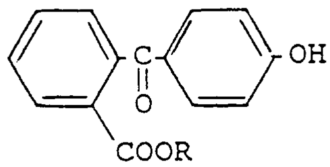

IV

in which R is represented by formula V

CH3
|
-C-CH3
|
H

V

is treated with a 2-piperidinoethyl derivative bearing a good leaving group.

2. A process for the production of compounds as claimed in claim 1, wherein a compound of the formula VI

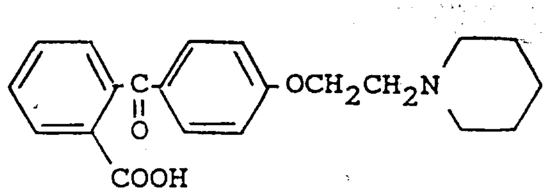

VI

is esterified with an alcohol of the formula VII

CH3
|
HO-C-CH3
|
H

VII

3. A process for the production of compounds as claimed in claim 2, wherein the carboxylate salt of the acid of formula VI is reacted with an alkyl halide.

4. A pharmaceutical preparation containing at least one compound as claimed in claim 1, optionally besides customary excipients and/or auxiliaries.

5. A pharmaceutical preparation as claimed in claim 4 for oral administration.

6. The use of a compound as claimed in claim 1 for the preparation of a pharmaceutical agent with spasmolytic activity.

**Patentansprüche**

**Patentansprüche für folgende Vertragsstaaten : AT, BE, CH, DE, FR, GB, IT, LI, LU, NL, SE**

1. Isopropyl-2-[4-(2-piperidin-ethoxy)-benzoyl]-benzoat und seine pharmazeutisch verträglichen Salze.

2. Verfahren zur Herstellung der Verbindung gemäß Anspruch 1, wobei eine Verbindung der Formel IV

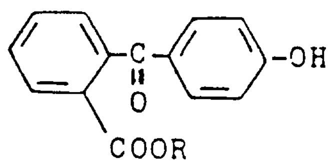

(IV)

in welcher R für die Formel V

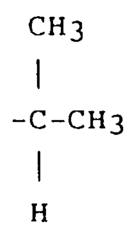

(V)

steht, mit einem 2-Piperidinethyl-Derivat behandelt wird, das eine gute Abgangs-Gruppe trägt.

3. Verfahren zur Herstellung von Verbindungen gemäß Anspruch 1, wobei eine Verbindung der Formel VI

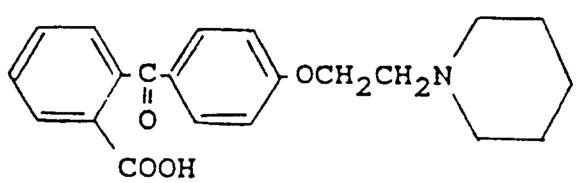

(VI)

mit einem Alkohol der Formel VII

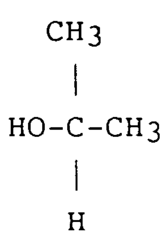

(VII)

verestert wird.

4. Verfahren zur Herstellung von Verbindungen gemäß Anspruch 1, wobei das Carboxylatsalz der Säure der Formel VI mit einem Alkylhalogenid umgesetzt wird.

5. Pharmazeutische Zubereitung, die die Verbindung gemäß Anspruch I enthält, wahlweise neben den üblichen Füll- und/oder Hilfsstoffen.

6. Pharmazeutische Zubereitung gemäß Anspruch 5 zur oralen Verabreichung.

7. Verwendung der Verbindung gemäß Anspruch 1 zur Herstellung eines pharmazeutischen Wirkstoffes mit spasmolytischer Wirksamkeit.

8. Verbindung gemäß Anspruch 1 als Arzneimittel.

**Patentansprüche für folgende Vertragsstaaten : ES, GR**

1. Verfahren zur Herstellung der Verbindung der Formel III

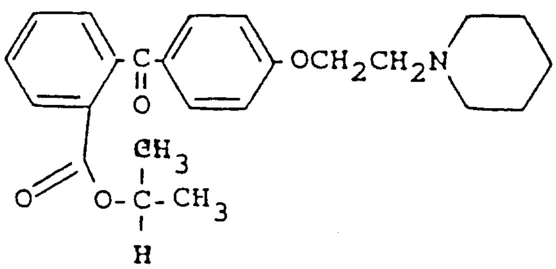

(III)

und ihrer pharmazeutisch verträglichen Salze, wobei eine Verbindung der Formel IV

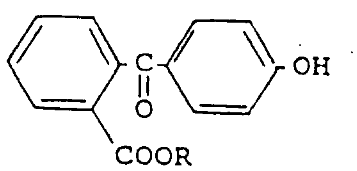

(IV)

in welcher R für die Formel V

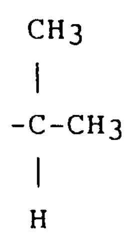

(V)

steht, mit einem 2-Piperidinethyl-Derivat behandelt wird, das eine gute Abgangs-Gruppe trägt.

2. Verfahren zur Herstellung von Verbindungen gemäß Anspruch 1, wobei eine Verbindung der Formel VI

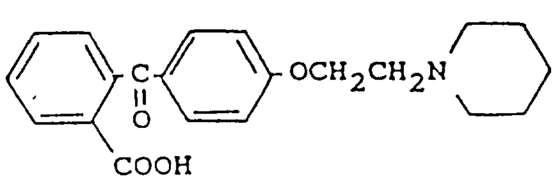

(VI)

mit einem Alkohol der Formel VII

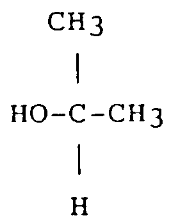

(VII)

verestert wird.

3. Verfahren zur Herstellung von Verbindungen gemäß Anspruch 2, wobei das Carboxylatsalz der Säure der Formel VI mit einem Alkylhalogenid umgesetzt wird.

4. Pharmazeutische Zubereitung, die mindestens eine Verbindung gemäß Anspruch 1 enthält, wahlweise neben den üblichen Füll- und/oder Hilfsstoffen.

5. Pharmazeutische Zubereitung gemäß Anspruch 4 zur oralen Verabreichung.

6. Verwendung der Verbindung gemäß Anspruch 1 zur Herstellung eines pharmazeutischen Wirkstoffes mit spasmolytischer Wirksamkeit.

**Revendications**
**Revendications pour les Etats contractants suivants : AT, BE, CH, DE, FR, GB, IT, LI, LU, NL, SE**

1. 2-[4-(2-pipéridinoéthoxy)benzoyl]benzoate d'isopropyle et sels pharmaceutiquement acceptables de celui-ci.

2. Procédé pour la préparation du composé selon la revendication 1, dans lequel on traite un composé de formule IV

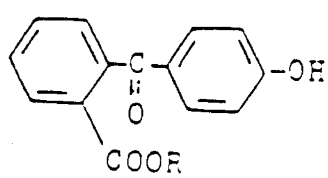

IV

dans lequel R est représenté par la formule V

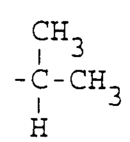

V

par un dérivé 2-pipéridinoéthyle portant un bon groupe partant.

3. Procédé pour la préparation de composés selon la revendication 1, dans lequel on estérifie un composé de formule VI

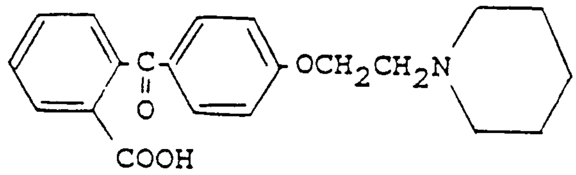

VI

par un alcool de formule VII

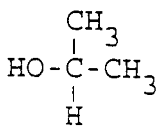

VII

4. Procédé pour la préparation de composés selon la revendication 1, dans lequel on fait réagir le carboxylate de l'acide de formule VI avec un halogénure d'alkyle.

5. Composition pharmaceutique contenant le composé selon la revendication 1, éventuellement en plus d'excipients et/ou agents auxiliaires usuels.

6. Composition pharmaceutique selon la revendication 5, pour administration orale.

7. Utilisation du composé selon la revendication 1, pour la préparation d'un agent pharmaceutique à activité spasmolytique.

8. Composé selon la revendication 1, en tant que produit pharmaceutique.

**Revendications pour les Etats contractants suivants : ES, GR**

1. Procédé pour la préparation d'un composé de formule III

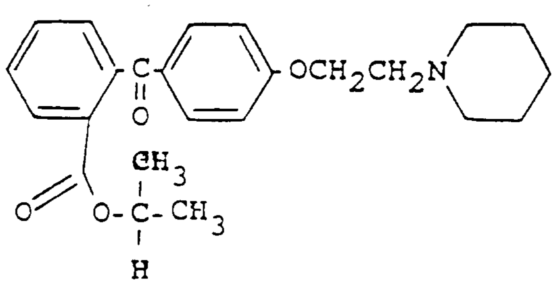

III

et de ses sels pharmaceutiquement acceptables, dans lequel on traite un composé de formule IV

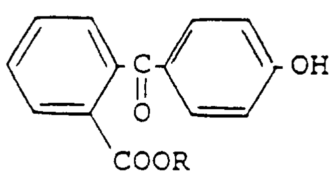

IV

dans lequel R est représenté par la formule V

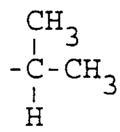

V

par un dérivé 2-pipéridinoéthylique portant un bon groupe partant.

2. Procédé pour la préparation de composés selon la revendication 1, dans lequel on estérifie un composé de formule VI

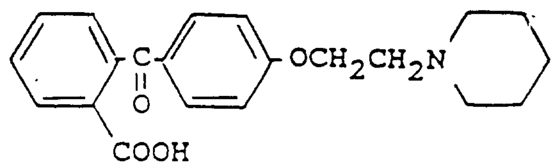

VI

par un alcool de formule VII

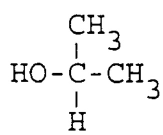

VII

3. Procédé pour la préparation de composés selon la revendication 1, dans lequel on fait réagir le carboxylate de l'acide de formule VI avec un halogénure d'alkyle.

4. Composition pharmaceutique contenant au moins un composé selon la revendication 1, éventuellement en plus d'excipients et/ou agents auxiliaires usuels.

5. Composition pharmaceutique selon la revendication 4, pour administration orale.

6. Utilisation du composé selon la revendication 1, pour la préparation d'un agent pharmaceutique à activité spasmolytique.

Control no drug

FIG.1

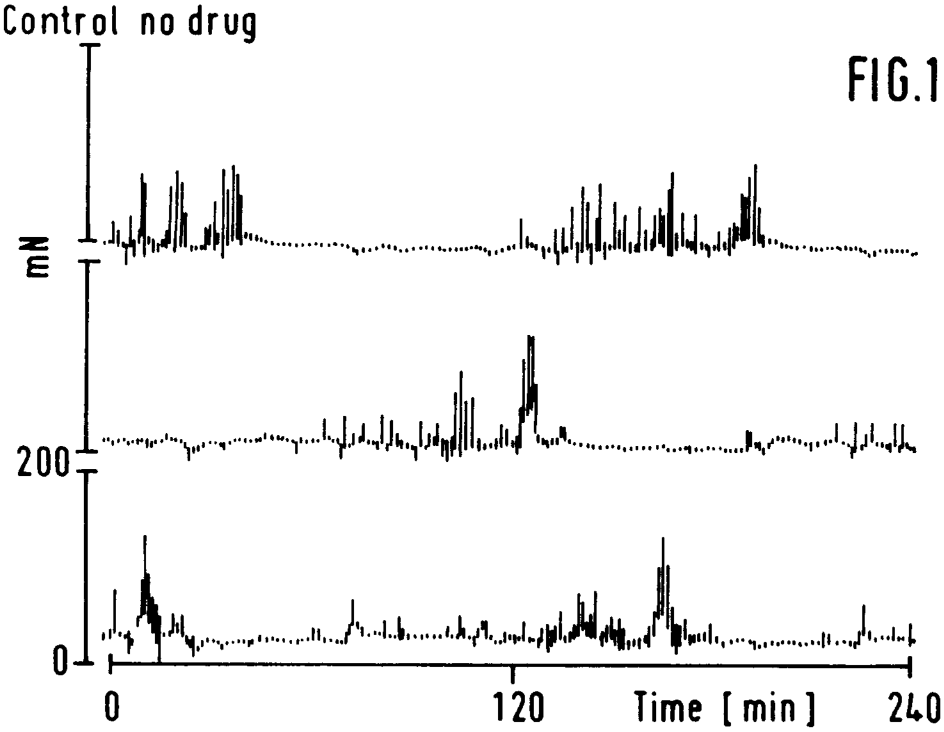


Effect of test compound II on small intestine motility in the dog

FIG.2

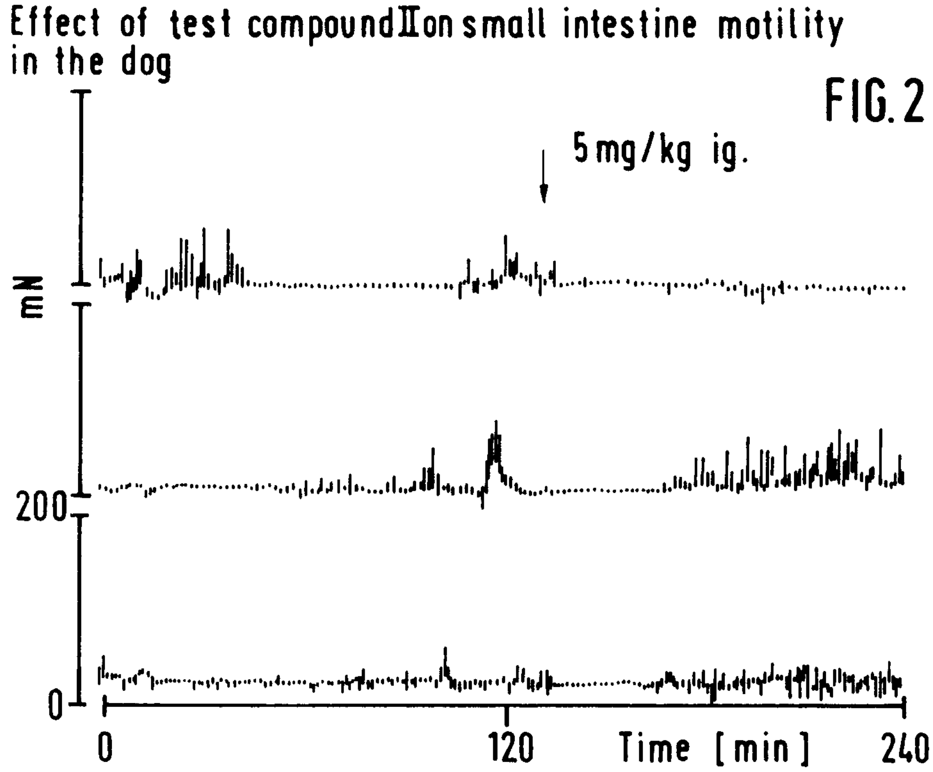

Inhibitory effect of test compound II 3 mg/kg ig. indicated by the arrow on colonic motility in the conscious dog.

FIG. 3